(19) Europäisches Patentamt
European Patent Office
Office européen des brevets

(11) Veröffentlichungsnummer: **0 089 920**
**B1**

(12) # EUROPÄISCHE PATENTSCHRIFT

(45) Veröffentlichungstag der Patentschrift: **09.04.86**

(51) Int. Cl.⁴: **C 07 D 249/08**, A 01 N 43/653 // C07F9/48

(21) Anmeldenummer: **83810063.4**

(22) Anmeldetag: **14.02.83**

(54) **Mikrobizide und wuchsregulierende Mittel.**

(30) Priorität: **19.02.82 CH 1049/82**

(43) Veröffentlichungstag der Anmeldung: **28.09.83 Patentblatt 83/39**

(45) Bekanntmachung des Hinweises auf die Patenterteilung: **09.04.86 Patentblatt 86/15**

(84) Benannte Vertragsstaaten: **AT BE CH DE FR GB IT LI NL SE**

(56) Entgegenhaltungen:
**ER-A-0 015 756**
**EP-A-0 047 057**
**DE-A-3 039 056**

(73) Patentinhaber: **CIBA-GEIGY AG, Klybeckstrasse 141, CH-4002 Basel (CH)**

(72) Erfinder: **Meyer, Alfred, Dr., St. Galler-Ring 220, CH-4054 Basel (CH)**
Erfinder: **Kunz, Walter, Dr., Im Goldbrunnen 55, CH-4104 Oberwil (CH)**
Erfinder: **Maier, Ludwig, Dr., Im Lee 28, CH-4144 Arlesheim (CH)**
Erfinder: **Rempfler, Hermann, Dr., Brückilsmattstrasse 16, CH-4107 Ettingen (CH)**

**EP 0 089 920 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue, substituierte Azolyl-olefinderivate der nachstehenden Formel I, sowie deren Säureadditionssalze, quaternäre Azoliumsalze und Metallkomplexe. Die Erfindung betrifft ferner die Herstellung dieser Substanzen sowie mikrobizide und wuchsregulierende Mittel, die als Wirkstoff mindestens eine dieser Verbindungen enthalten. Die Erfindung betrifft auch die Herstellung der genannten Mittel und die Verwendung der Wirkstoffe oder der Mittel zur Regulierung des Pflanzenwachstums und zur Bekämpfung von schädlichen Mikroorganismen.

Es werden hierin Verbindungen der allgemeinen Formel I

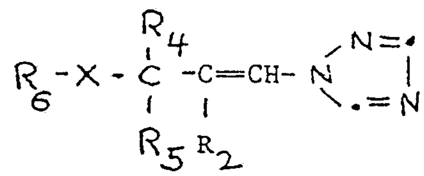

(I)

umfasst, worin

$R_2$ für $C_1$-$C_{12}$-Alkyl, $C_3$-$C_8$-Cycloalkyl, durch gegebenenfalls substituiertes Phenyl, $C_1$-$C_4$-Alkoxycarbonyl oder $C_2$-$C_6$-Alkenyl substituiertes $C_1$-$C_4$-Alkyl oder ein- bis vierfach durch $C_1$-$C_4$-Alkyl substituiertes $C_3$-$C_8$-Cycloalkyl steht;

$R_4$ und $R_5$ unabhängig voneinander für Wasserstoff oder $C_1$-$C_4$-Alkyl stehen;

X Sauerstoff oder Schwefel bedeutet;

$R_6$ für einen unsubstituierten oder ein- oder mehrfach substituierten Rest steht, ausgewählt aus der Reihe: Phenyl, Naphthyl, Biphenyl, Benzylphenyl, Benzoxyphenyl, Phenoxyphenyl und Aralkyl, wobei die Substituenten ausgewählt sind aus der Reihe: Halogen, Cyano, $C_1$-$C_4$-Alkyl, $C_1$-$C_5$-Alkoxy, $C_1$-$C_5$-Haloalkoxy, $C_1$-$C_3$-Alkylthio, $C_1$-$C_3$-Haloalkyl, und Nitro,

bedeuten; unter Einschluss ihrer Säureadditionssalze, quaternären Azoliumsalze und Metallkomplexe.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, Undecyl, Dodecyl usw. sowie ihre Isomeren, wie z.B. Isopropyl, Isobutyl, tert.-Butyl, Isopentyl usw.. Haloalkyl steht für einen dreifach bis perhalogenierten Alkylsubstituenten, wie z.B. $CHCl_2$,$CF_3$ $CHF_2$, $CH_2Cl$, $CCl_3$, $CH_2F$, $CH_2CH_2Cl$, $CHBr_2$ usw. Unter Halogen soll hier und im folgenden Fluor, Chlor, Brom oder Jod, vorzugsweise Fluor, Chlor oder Brom, verstanden werden. Naphthyl steht für α- oder β-Naphthyl, vorzugsweise α-Naphthyl. Der Ausdruck Haloalkoxy oder Haloalkylthio steht für einen Alkoxy- oder Alkylthiorest dessen Haloalkylanteil wie weiter oben unter Haloalkyl definiert ist, Alkenyl bedeutet z.B. Propenyl-(1), Allyl, Butenyl-(1), Butenyl-(2) oder Butenyl-(3), Alkinyl steht z.B. für Propionyl-(1) oder Propargyl. Aryl bedeutet z.B. Naphthyl, insbesondere Phenyl und Aralkyl einen Niederalkylrest, der durch eine der obigen Arylgruppen substituiert ist. Cycloalkyl bedeutet je nach Zahl der Kohlenstoffatome z.B. Cyclopropyl Cyclobutyl Cyclopentyl Cyclohexyl Cycloheptyl, Cyclooctyl usw.

Die vorliegende Erfindung betrifft somit die freien Verbindungen der Formel I, deren Säureadditionssalze, quaternären Azoliumsalze und Metallkomplexe. Die freien Verbindungen sind bevorzugt.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden enthalten. Komplexe mit den Metallen Kupfer, Zink, Mangan und Zinn sind bevorzugt.

Die Verbindungen der Formel 1 sind bei Raumtemperatur stabile Feststoffe, oder überwiegend Oele oder Harze die sich durch sehr wertvolle mikrobizide und wuchsregulierende Eigenschaften auszeichnen. Sie lassen sich auf dem Agrarsektor oder verwandten Gebieten präventiv und kurativ zur Bekämpfung von pflanzenschädigenden Mikroorganismen und zur Regulierung des Pflanzenwuchses einsetzen, dabei sind die Triazolylmethylderivate im Umfang der Formel I bevorzugt. Die erfindungsgemässen Wirkstoffe der Formel I zeichnen sich durch eine sehr gute Verträglichkeit bei Kulturpflanzen aus.

Aufgrund ihrer ausgeprägten wuchsregulierenden und/oder mikrobiziden Wirkung sind z.B. folgende Wirkstoffgruppen bevorzugt:

a) Verbindungen der Formel I, worin
$R_2$ $C_1$-$C_9$-Alkyl bedeutet;
wobei $R_4$ und $R_5$ unabhängig voneinander für Wasserstoff oder $C_1$-$C_4$-Alkyl stehen; X Sauerstoff bedeutet und $R_6$ für gegebenenfalls durch Halogen, $CF_3$ oder $C_1$-$C_3$-Alkyl substituierten Phenyl steht.

b) Verbindungen der Formel I,
$R_2$ tert-Butyl oder iso-Propyl bedeutet;
$R_4$ Wasserstoff und $R_5$ $C_1$-$C_4$-Alkyl bedeutet; X für Sauerstoff steht und $R_6$ gegebenenfalls durch Chlor, Brom, Fluor, $CF_3$ oder Methyl substituiertes Phenyl steht.

Als Einzelsubstanzen sind insbesondere folgende Verbindungen bevorzugt.
1-(1H-1,2,4-Triazol-1-yl)-2-(4-chlorphenoxymethyl)-3,3-dimethylbuten,
1-(1H-1,2,4-Triazol-1-yl)-2-(iso-propyl)-3-(4-chlorphenoxy)-penten,
1-(1H-1,2,4-Triazol-1-yl)-2-(tert.-butyl)-3-(4-chlorphenoxy)-hepten,
1-(1H-1,2,4-Triazol-1-yl)-2-(tert.-butyl)-3-(4-fluorphenoxy)-buten,
in Form der Isomeren-Gemische sowie als reine E- oder Z-Isomeren.

Die Verbindungen der Formel I können nach einer ganzen Reihe von Reaktionsvarianten i bis iv, wie anschliessend in einem Reaktionsschema skizziert und darauffolgend im einzelnen aufgeführt, hergestellt werden. In den Formeln I, II, III, IV, V, VI, VII, VIII, IX, X, XI, XII, XIII, XIV und XV haben die Substituenten $R_1$, $R_2$ und $R_3$ die unter Formel I angegebenen Bedeutungen. $R_{10}$ steht für Phenyl oder $C_1$-$C_4$-Alkyl, $R_{11}$ für Phenyl oder gegebenenfalls durch Hydroxyl substituiertes $C_1$-$C_4$-Alkyl. A steht stellvertretend für eine der üblichen Abgangsgruppen, nämlich für Chlor, Brom, Jod oder die Gruppen -OCO-$R_{12}$ oder -OSO-$R_{12}$, wobei $R_{12}$ $C_1$-$C_3$-Alkyl, $C_1$-$C_3$-Halo-alkyl oder gegebenenfalls durch Halogen, Methyl, Nitro, Trifluormethyl oder Methoxy substituiertes Phenyl bedeutet, vorzugsweise jedoch für Chlor oder Brom. Der Ausdruck Hal steht für Halogen, vorzugsweise für Fluor, Chlor oder Brom. M repräsentiert ein Alkalimetall, insbesondere Natrium oder Kalium.

**REAKTIONS-SCHEMA**

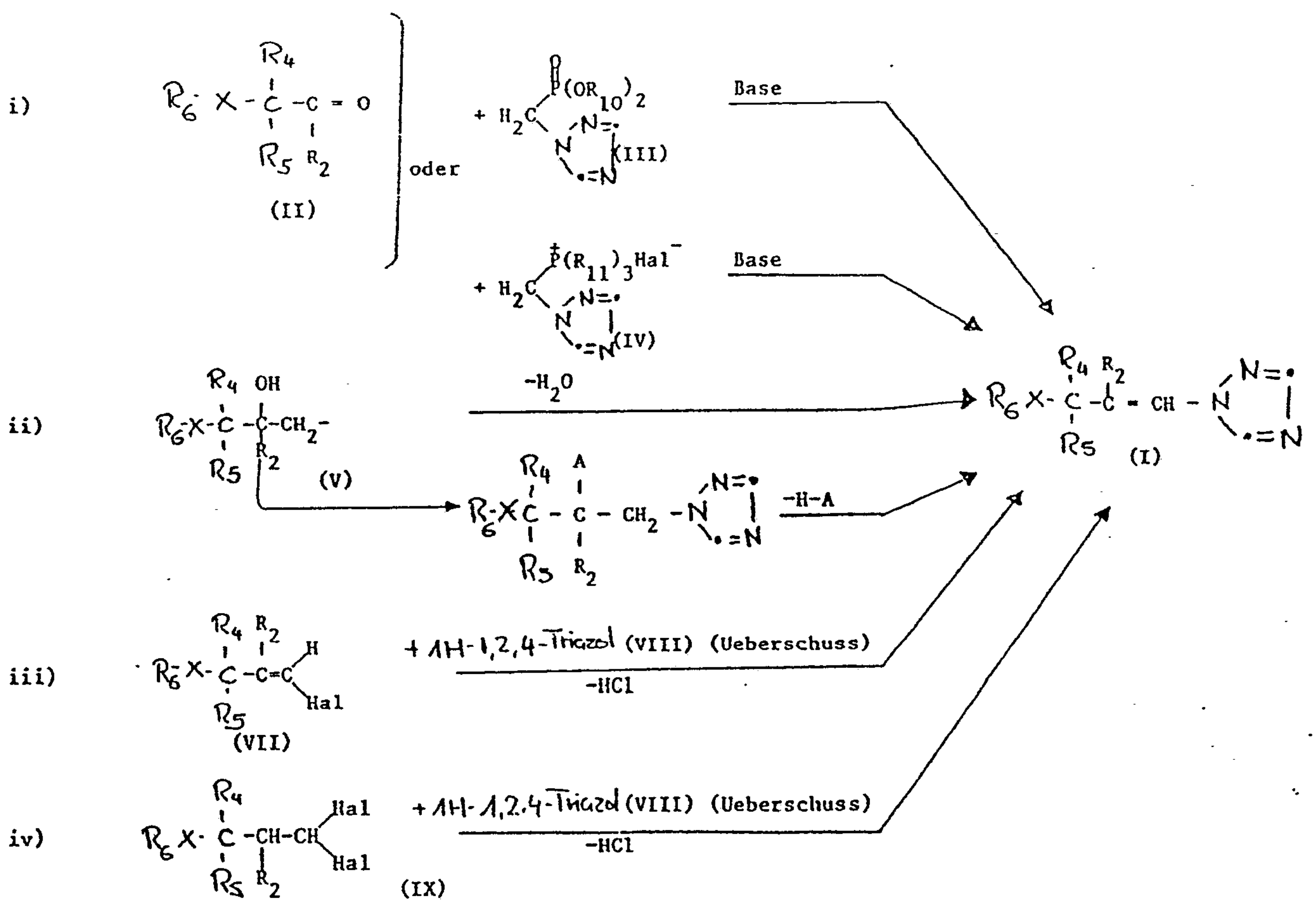

Zur Herstellung der Verbindungen der Formel I kann man im einzelnen wie folgt vorgehen:
i) Azolyl-olefin-derivate der Formel I können hergestellt werden, indem man ein Keton der Formel II

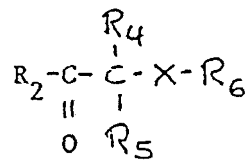 (II),

worin $R_2$ und $R_3$ die unter Formel 1 angegebenen Bedeutungen haben, durch Umsetzung mit einem 1H-Azol-1-yl-methylphosphonat der Formel III

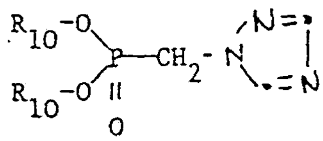 (III),

worin die Reste $R_{10}$ unabhängig voneinander Phenyl oder $C_1$-$C_4$-Alkyl bedeuten oder mit einem Phosphoniumsalz der Formel IV

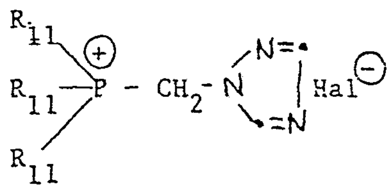 (IV),

worin die Reste $R_{11}$ unabhängig voneinander Phenyl oder gegebenenfalls durch Hydroxyl substituiertes $C_1$-$C_4$-Alkyl bedeuten $R_1$ für eine Azolgruppe steht und Hal für Chlor, Brom steht, in einem reaktionsinerten Lösungsmittel in Gegenwart einer starken Base umsetzt.

Die Ueberführung der Ketone der Formel II in die Azolyl-olefinderivate der Formel I kann somit nach der Verfahrensvariante entweder unter Verwendung der Phosphonate der Formel III oder der Phosphoniumsalze der Formel IV erfolgen.

Die Reaktionsvariante, die als Reaktionspartner die Phosphonate der Formel III verwendet, wird vorteilhafterweise so geführt, dass man das Phosphonat zunächst mit einer starken Base und danach mit dem Keton der Formel II versetzt.

Als Lösungsmittel bei dieser Eintopfreaktion können inerte, polare, aprotische Lösungsmittel verwendet werden. Solche Lösungsmittel sind Ether wie Diethylether, Tetrahydrofuran, Dioxan, Dimethoxyethan oder Diethylenglykol-dimethylether; Säureamide wie Dimethylformamid, 2-Pyrrolidinon oder Hexamethylphosphorsäuretriamid; und Sulfoxide wie Dimethylsulfoxid.

Bevorzugt sind Lösungsmittel, deren Siedepunkt über +60°C liegt, also z.B. Tetrahydrofuran, Dioxan, Dimethoxyethan, Dimethylformamid oder Dimethylsulfoxid.

Als starke Basen können verwendet werden: metallorganische Verbindungen wie Methyllithium, Propyllithium, Butyllithium, Phenyllithium; oder Triphenylmethylnatrium; Alkoholate wie Natriummethylat, Natriumethylat, Kaliumethylat oder Kalium-tert.-butylat; Metallhydride wie Lithiumhydrid, Natriumhydrid oder Calciumhydrid; und Alkaliamide wie Natriumamid oder Lithiumdiisopropylamid.

Als bevorzugte Basen sind die Metallhydride, die metallorganischen Verbindungen und die Alkoholate zu verstehen.

Da es sich sowohl bei der Umsetzung des Phosphonats III mit der starken Base zum entsprechenden Salz, als auch bei der weiteren Reaktion mit dem Keton II um exotherme Vorgänge handelt, kühlt man das Reaktionsgefäss jeweils vor dem Zusetzen des Reagenzes ab und erwärmt dann anschliessend zur Vervollständigung der Umsetzung. Geeignete Temperaturintervalle sind für die erste Stufe -40 bis +40°C, insbesondere -20° bis +40°C und für die zweite Stufe -20° bis +80°C, insbesondere 0° bis +60°C. Man kann jedoch auch die Base bei 0-60° vorgelegen und das Gemisch aus Keton II und Phosphonat III zudosieren.

Das entstandene Azolyl-olefin-derivat der Formel I wird isoliert, indem man das Reaktionsgemisch mit Wasser versetzt, einem mit Wasser nicht mischbaren Lösungsmittel extrahiert und die organischen Phasen eindampft.

Die Reaktionsvariante, die als Reaktionspartner die Phosphoniumsalze der Formel IV verwendet, wird vorteilhafterweise so geführt, dass man das Phosphoniumsalz zunächst mit einer starken Base und danach mit dem Keton der Formel II versetzt.

Es hat sich auch hier als günstig erwiesen, wenn man die Base im Ueberschuss zusetzt. Ueblicherweise beträgt der Ueberschuss weniger als das Doppelte der zur Reaktion benötigten molaren Menge, also zwischen 0,01 und 1 Aequivalenten.

Als Lösungsmittel bei dieser Eintopfreaktion können wiederum inerte, polare, aprotische Lösungsmittel verwendet werden. Solche Lösungsmittel sind Ether wie Diethylether, Tetrahydrofuran, Dioxan, Dimethoxyethan oder Diethylenglykol-dimethylether; Säureamide wie Dimethylformamid, 2-Pyrrolidinon oder Hexamethylphosphorsäuretriamid; und Sulfoxide wie Dimethylsulfoxid.

Bevorzugt sind Lösungsmittel, deren Siedepunkt über 80°C liegt, also z.B. Dioxan, Dimethoxyethan, Diethylenglykol-dimethylether, Dimethylformamid, 2-Pyrrolidinon oder Dimethylsulfoxid.

Als starke Basen können verwendet werden: metallorganische Verbindungen wie Methyllithium, Propyllithium, Butyllithium, Phenyllithium, oder Triphenylmethylnatrium; Alkoholate wie Natriummethylat, Natriumethylat, Kaliumethylat oder Kalium-tert.-butylat; Metallhydride wie Lithiumhydrid, Natriumhydrid oder Calciumhydrid; und Alkaliamide wie Natriumamid, oder Lithiumdiisopropylamid.

Als bevorzugte Basen sind die Metallhydride und die metallorganischen Verbindungen zu verstehen.

Da es sich sowohl bei der Umsetzung des Phosphoniumsalzes mit der starken Base zum entsprechenden Ylid, als auch bei der weiteren Reaktion mit dem Keton II um exotherme Vorgänge handelt, kühlt man das Reaktionsgefäss jeweils vor dem Zusetzen des Reagenses ab und erwärmt dann anschliessend zur Vervollständigung der Umsetzung. Geeignete Temperaturintervalle sind für die erste Stufe − 40° bis +80°C, insbesondere − 20° bis +40°C und für die zweite Stufe − 20° bis +110°C, insbesondere 0° bis +60°C.

Das entstandene Azolyl-olefin-derivat der Formel I wird isoliert, indem man das Reaktionsgemisch mit Diethylether verdünnt, das ausgefallene Phosphinoxid abfiltriert und das Filtrat eindampft. Um am Phosphinoxid anhaftendes Produkt zu gewinnen, kann es notwendig sein, den Filterkuchen mehrmals mit Ether auszuwaschen.

In den Fällen, in denen man bei der Herstellungsvariante i von Salzen der Formeln III oder IV ausgeht, kann sich der Zusatz eines Kronenethers als sehr vorteilhaft erweisen. So ist beispielsweise die Verwendung von 18-Krone-6 und 15-Krone-5 sehr günstig.

Bei der Herstellungsvariante i entstehen üblicherweise Gemische von cis- und trans-Olefinen, in denen der cis-Olefinanteil im allgemeinen überwiegt. Die Bildung eines höheren trans-Olefin-anteils kann durch einen grösseren Ueberschuss am Ylid oder durch Zusatz von Lithiumsalzen (z.B. $LiClO_4$) erzielt werden. [Vgl. M. Schlosser, Chem. Ber. 103, 2841]. Die einzelnen Isomeren (cis- resp. trans-Olefine) können auch durch übliche physikalische Methoden (z.B. Chromatographie) getrennt werden und unterschiedliche biologische Aktivitäten aufweisen.

ii) Azolyl-olefin-derivate der Formel I können ferner hergestellt werden, indem man entweder direkt aus einer Verbindung der Formel V

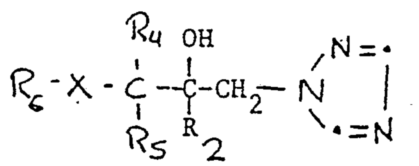 (V),

worin $R_1$, $R_2$ und $R_3$ die unter Formel I angegebenen Bedeutungen haben, mit einem wasserentziehenden Mittel Wasser abspaltet; oder indem man vorteilhafterweise die Verbindung der Formel V durch Austausch der freien Hydroxylgruppe gegen eine der üblichen Abgangsgruppen A zuerst in eine Verbindung der Formel VI überführt

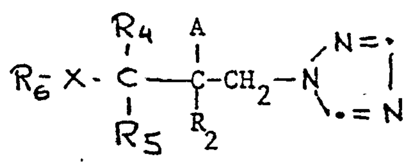 (VI)

und VI dann durch Abspaltung von H-A in die Endprodukte der Formel I verwandelt. Unter einer üblichen Angangsgruppe A sind im Rahmen vorliegender Erfindung die Substituenten Chlor, Brom, Jod sowie die Gruppen $-OCO-R_{12}$ und $-OSO-R_{12}$ zu verstehen, wobei $R_{12}$ für $C_1$-$C_3$-Alkyl, $C_1$-$C_3$-Haloalkyl oder gegebenenfalls durch Halogen, Methyl, Nitro, Trifluormethyl oder Methoxy substituiertes Phenyl steht, vorzugsweise jedoch für Chlor oder Brom.

Die Reaktion kann in vielen Fällen im sogenannten Eintopfverfahren durchgeführt werden, d.h. das Zwischenprodukt der Formel VI kann zwar, muss aber nicht, aus dem Reaktionsmedium isoliert werden.

Der Wasserentzug aus Verbindungen der Formel V erfolgt zweckmässigerweise in einem üblichen reaktionsinerten Lösungsmittel oder Lösungsmittelgemisch. Geeignete Lösungsmittel sind z.B. Alkohole, wie Niederalkanole (Methanol, Ethanol, n-Propanol, iso-Propanol, n-Butanol, Amylalkohol usw.); Ether und etherartige Verbindungen, wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Xylol, Petrolether; halogenierte Kohlenwasserstoffe wie Chlorbenzol, Methylenchlorid, Ethylenchlorid, Chloroform, Tetrachlorkohlenstoff, Tetrachlorethylen usw. Als wasserentziehende Mittel kommen z.B. starke Säuren, insbesondere konzentrierte oder verdünnte Mineralsäuren wie Phosphorsäure, Schwefelsäure oder

Halogenwasserstoffsäuren (Chlorwasserstoff-, Bromwasserstoff-, Jodwasserstoff- oder Fluorwasserstoffsäure in Frage. Die Reaktion wird bei Temperaturen von 0° bis +180°C durchgeführt, üblicherweise bei erhöhter Temperatur. Als wasserentziehendes Mittel können auch Carbodiimide wie N,N'-Dicyclohexylcarbodiimid eingesetzt werden. Hier liegen die Reaktionstemperaturen bei 0° bis +150°C.

Der Austausch der freien Hydroxylgruppe in den Verbindungen der Formel V gegen eine Abgangsgruppe A wird bevorzugt in einem reaktionsinerten Lösungsmittel durchgeführt. Beispiele solcher Lösungsmittel sind: Aromatische und aliphatische Kohlenwasserstoffe wie Benzol, Toluol, Xylole, Petrolether, Ligroin oder Cyclohexan; halogenierte Kohlenwasserstoffe wie Chlorbenzol, Methylenchlorid, Ethylenchlorid, Chloroform, Tetrachlorkohlenstoff oder Tetrachlorethylen; Ether und etherartige Verbindungen wie Diethylether, Diisopropylether, T-Butylmethylether, Dimethoxyethan, Dioxan, Tetrahydrofuran oder Anisol; Ester wie Ethylacetat, Propylacetat oder Butylacetat; Nitrile wie Acetonitril; oder Verbindungen wie Dimethylsulfoxid, Dimethylformamid und Gemische solcher Lösungsmittel untereinander.

Die Einführung der Abgangsgruppen A erfolgt nach üblichen Methoden. Bedeutet A Chlor oder Brom, so wird als Reagenz z.B. Phosphoroxychlorid, Phosphortrichlorid, Phosphorpentachlorid oder das entsprechende Bromid oder vorzugsweise Thionylchlorid eingesetzt. Man arbeitet im allgemeinen bei Temperaturen von 0° bis +120°C. Im Falle von A = Brom verwendet man bevorzugt Phosphortribromid oder Phosphorpentabromid und führt die Reaktion bei 0° bis +50°C durch. Steht A für eine der Gruppen -OCO-R oder $-OSO_2-R$ so wird als Reagenz üblicherweise das entsprechende Säurehalogenid, insbesondere Säurechlorid, eingesetzt. Hierbei ist es zweckmässig, wenn die Reaktion bei Temperaturen von -20° bis +50°C, vorzugsweise -10° bis +30°C, und in Gegenwart einer schwachen Base wie Pyridin oder Triethylamin durchgeführt wird.

Zur Abspaltung der hydrierten Abgangsgruppe (H-A) wird eine geeignete Base zugesetzt. Beispiele derartiger Basen sind: tertiäre Amine (Triethylamin, Ethyldiisopropylamin etc.); bicyclische Amine wie 1,4-Diazabicyclo[2.2.2]octan, 1,5-Diazabicyclo]3.4.0]-nonen-5 usw.; Dialkylaniline wie N,N-Dimethylanilin, N,N-Methylethylanilin usw.; heterocyclische Basen wie Pyridin, Collidin, Chinolin usw.; ferner anorganische Basen wie Natriumacetat, Natriumhydrogencarbonat, Alkali– und Erdalkalihydroxide [NaOH, KOH, $Ca(OH_2$, $Ba(OH)_2$]; Alkalialkoholate [Natrium- oder Kaliumethanolat, Kaliumtertiärbutylat] usw. Die Umsetzung erfolgt bei einem Temperaturbereich von 0° bis +120°C.

iii) Bei einer anderen Herstellungsvariante für Verbindungen der Formel I geht man von Chlor-olefin-derivaten der Formel VIII aus

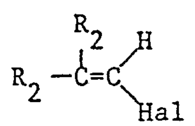

(VII),

worin $R_2$ und $R_3$ die unter Formel I angegebenen Bedeutungen haben, der Substituent Hal für eines der Halogenatome, insbesondere für Fluor, Chlor oder Brom stehen, und setzt diese in der Schmelze bei +150° bis +350°C und gegebenenfalls unter Druck mit überschüssigem 1H- 1,2,4- Triazol um.

Man verwendet hierbei bis zu zehnfachen Ueberschuss an Triazol bevorzugt jedoch seine dreifache Menge, bezogen auf die Verbindung der Formel VII.

iv) Verbindungen der Formel I lassen sich ferner aus Verbindungen der Formel IX herstellen

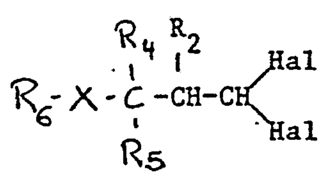

(IX),

worin $R_2$ und $R_3$ die unter Formel I angegebenen Bedeutungen haben, und die Substituenten Hal unabhängig voneinander für Halogen, insbesondere für Fluor, Chlor oder Brom stehen, indem man letztere in einem dipolaren aprotischen Lösungsmittel, gegebenenfalls in Gegenwart einer Base, bei +40° bis +180°C, vorzugsweise +80° bis +120°C, oder in der Schmelze bei +150° bis +350°C mit überschüssigem 1H-1,2,4 - Triazól herstellt.

Die Base kann im Verlauf des Schmelzvorganges in das Reaktionsgemisch eingetragen werden. Falls erforderlich, wird unter Druck (bis 15 bar) gearbeitet. Als Basen kommen insbesondere anorganische Basen wie Alkali- und Erdalkalihydroxide (KOH, NaOH) in Frage. Geeignete aprotische Lösungsmittel sind beispielsweise Dimethylformamid, Dimethylsulfoxid, Hexamethylphosphorsäuretriamid und Nitrile wie Acetonitril.

Die Ausgangsverbindungen der Formel II sind zum Teil bekannt und im Handel erhältlich, oder sie können analog zu den bekannten Spezies leicht hergestellt werden. Die 1H-1,2,4-Triazol-1-yl-methylphosphonate der Formel III lassen sich dadurch herstellen, dass man ein 1-Halogenmethyl-1H-Azol der Formel X

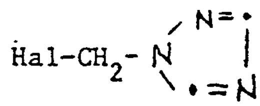 (X),

worin Hal für Chlor, Brom oder Jod steht, entweder mit einem sekundären Phosphit der Formel XI

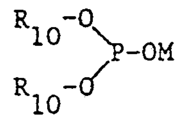 (XI),

worin die Reste $R_{10}$ unabhängig voneinander Phenyl oder $C_1$-$C_4$-Alkyl bedeuten und M für ein Alkalimetallatom steht, oder mit einem tertiären Phosphit der Formel XII umsetzt

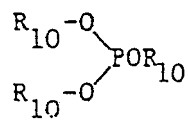 (XII),

worin $R_{10}$ die unter Formel (XI) angegebenen Bedeutungen hat.

Mit Vorteil wird das Verfahren zur Herstellung der Phosphonate der Formel III in einem inerten organischen Lösungsmittel durchgeführt. Solche Lösungsmittel sind aromatische Kohlenwasserstoffe wie Benzol, Toluol oder Xylol; Ether wie Diethylether, Tetrahydrofuran, Dioxan, Dimethoxyethan oder Diethylenglykol-dimethylether oder Nitrile wie Acetonitril. Verwendet man ein sekundäres Alkaliphosphit der Formel XI, ist es angebracht, ein polares Lösungsmittel wie Acetonitril, Dimethoxyethan oder Diethylenglykol-dimethylether einzusetzen. Wird zur Syntnese der Phosphonate ein tertiäres Phosphit der Formel VII eingesetzt, so kann dieses selbst häufig als Lösungsmittel dienen.

In jedem Fall jedoch empfiehlt es sich, die Reaktionsmischung zu erwärmen; bei der sekundär-Phosphitreaktion auf 50° bis 150°C, vorzugsweise auf +80° bis +120°C und bei der tertiär-Phosphitreaktion auf +100° bis +180°C, vorzugsweise auf +120° bis +160°C.

Bei den sekundären Alkaliphosphiten handelt es sich im allgemeinen um Natrium- oder Kaliumphosphite.

Die Phosphite der Formel III werden isoliert, indem man gegebenenfalls den entstandenen Niederschlag abtrennt, die Lösung eindampft und den Rückstand destilliert.

Die 1H-1,2,4-Triazol-1-yl-methylphosphoniumsalze der allgemeinen Formel IV lassen sich herstellen, indem man ein 1-Halogenmethyl-1H-1,2,4- Triazol mit einem Phosphin der Formel XIII umsetzt

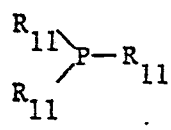 (XIII),

worin $R_{11}$die unter Formel IV angegebenen Bedeutungen hat.

Mit Vorteil wird das Verfahren zur Herstellung der Phosphoniumsalze der Formel IV in einem inerten, organischen Lösungsmittel durchgeführt. Solche Lösungsmittel sind aromatische Kohlenwasserstoffe wie Benzol, Toluol oder Xylol; Ether wie Diethylether, Tetrahydrofuran, Dioxan, Dimethoxyethan oder Diethylenglykol-dimethylether; Säureamide wie Dimethylformamid oder 2-Pyrrolidinon oder Nitrile wie Acetonitril. Vorzugsweise werden polare Lösungsmittel wie Dimethylformamid, Acetonitril oder Dimethoxyethan verwendet.

Es empfiehlt sich, das Reaktionsgemisch auf +30° bis +120°C, vorzugsweise auf +50° bis +100°C, zu erwärmen.

Beim Abkühlen der Reaktionsmischung kristallisieren die Phosphoniumsalze meist in reiner Form aus, so dass sich ein Extraktions- oder Fällungsverfahren zur Isolation dieser Verbindungen im allgemeinen erübrigt. Fällt das Produkt nicht direkt an, so kann es durch einfaches Abdampfen des Lösungsmittels erhalten werden.

Einige der Phosphoniumsalze der Formel IV haben selbst auch fungizide Eigenschaften und sind, sofern sie neu sind, ein Teil der Erfindung.

Die 1-Halogenmethyl-1H-azole der Formel X

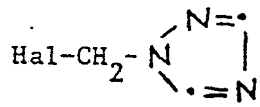 (X),

worin Hal für Chlor, Brom oder Jod steht, werden erhalten, indem man 1-Hydroxymethyl-1H-azol der Formel XIV

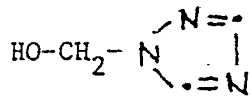 (XIV)

mit einem Halogenierungsmittel umsetzt und die entstandenen Hydrohalogenide der Formel XV

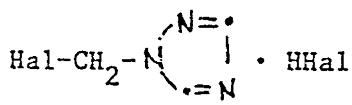 (XV),

worin Hal die unter Formel X angegebene Bedeutung hat, mit einer Base behandelt.

Als Basen eignen sich starke anorganische Basen, z.B. Hydroxide wie Natrium- und Kaliumhydroxid.

Als Halogenierungsmittel können Verwendung finden: Phosgen, Thionylchlorid, Thionylbromid, Phosphortrichlorid, Phosphortribromid, Phosphorpentachlorid, Phosphoroxychlorid oder Jodwasserstoffsäure. Vorzugsweise werden Thionylchlorid und Thionylbromid zur Halogenierung eingesetzt, da die entstehenden Nebenprodukte gasförmig sind, aus der Reaktionslösung entweichen und deshalb die Reaktion nicht beeinflussen. Die Jodverbindungen werden mit Vorteil aus bereits halogenierten Verbindungen erhalten, wenn man diese mit Jodwasserstoffsäure umsetzt. Eine weitere Methode zur Herstellung von Chloriden, Bromiden und Jodiden besteht in der Umsetzung von XIV mit den entsprechenden Trialkylsilyl-Halogeniden.

Die Halogenierungsreaktion wird in inerten Lösungsmitteln ausgeführt, wie z.B. Kohlenwasserstoffen wie Hexan, Cyclohexan, Benzol, Toluol oder Xylol oder Ethern wie Diethylether, Tetrahydrofuran, Dioxan oder Dimethoxyethan. Bei Verwendung von flüssigen Halogenierungsmitteln kann häufig ganz auf ein Lösungsmittel verzichtet werden. Die Reaktion wird dann in einem Ueberschuss des Reagenzes, z.B. Thionylchlorid chlorid oder -bromid, durchgeführt.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid. Ferner kann das Arbeiten unter erhöhtem Druck günstig auf die Ausbeute auswirken. Die Ausgangsprodukte der Formel V sind grösstenteils bekannt oder werden analog zu beschriebenen Methoden hergestellt (vgl. GB-PS 2,064,520).

Verbindungen des Typs der Formel VII sind ebenfalls aus der EP-PS 0,004,315 bekannt oder können nach den dort beschriebencn Methoden hergestellt werden. Auch Verbindungen des Typs der Formel IX sind aus der Literatur bekannt oder lassen sich analog zu den beschriebenen Verfahren herstellen [vgl. JACS 67, 1591 (1945)]. Die Ausgangsverbindungen der Formeln VIII, XI, XII, XIII und XIV sind allgemein bekannt, sie stellen im allgemeinen Grundchemikalien dar und können nach an sich bekannten Methoden hergestellt werden. Sie sind grösstenteils im Handel erhältlich.

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen

in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z.B. mehr oder grössere Früchte zur Ausbildung kommen.

Ertragssteigerungen können in manchen Fällen auch durch einen Eingriff in den pflanzlichen Stoffwechsel erreicht werden, ohne dass sich Aenderungen des vegetativen Wachstums bemerkbar machen. Wachstumsregulatoren können ferner eine Veränderung der Zusammensetzung der Pflanzen bewirken,so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen, z.B. um bei Tabakpflanzen nach der Dekapitierung die Ausbildung von Seitentrieben zu verhindern und damit das Blattwachstum zu fördern.

Durch Einsatz von Wachstumsregulatoren lässt sich der vorzeitige Fruchtfall verhindern. Es ist jedoch auch möglich, den Fruchtfall, - zum Beispiel bei Obst -, im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden Voraussetzungen dafür geschaffen, dass z.B. bei Tabak, Tomaten oder Kaffee, eine vollständige mechanische oder manuelle Beerntung in nur einem Arbeitsgang vorgenommen werden kann.

Durch die Anwendung von Wachstumsregulatoren kann auch die Samenoder Knospenruhe der Pflanzen,also die endogene Jahresrhythmik, beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z.B. um in frostgefährdeten Gebieten eine Schädigung durch Spätfröste zu vermeiden. Andererseits gelingt es, das Wurzelwachstum zu und/oder die Ausbildung von Sprösslingen zu stimulieren, so dass das Wachstum auf eine kürzere Zeitdauer beschränkt werden kann. Wachstumsregulatoren können auch eine Halophilie bei den Kulturpflanzen erzeugen. Damit werden die Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Unter dem Einfluss von Wachstumsregulatoren kann das Altern (dia Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso kann durch Behandlung von Kulturpflanzen über eine Verlängerung der Phase photosynthetischer Aktivität eine beachtliche Ertragssteigerung erzielt werden.

Ein weiteres wichtiges Anwendungsgebiet für Wuchshemmer ist deren Einsatz zur Hemmung eines

übermässigen Wachstums bei tropischen Bodenbeckungspflanzen, den sogenannten Cover crops. In tropischen und subtropischen Monokulturen, wie z.B. in Palmplantagen, Baumwoll-, Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der Austrocknung, Versorgung mit Stickstoff) und zur Verhinderung von Erosion (Abtragung durch Wind und Wasser) dienen. Durch Applikation der erfindungsgemässen Wirkstoffe kann nunmehr das Wachstum dieser Cover crops kontrolliert und somit die Wuchshöhe dieser Bodenbedeckungspflanzen auf einem niedrigen Niveau gehalten werden, so dass ein gesundes Gedeihen der Kulturpflanzen und die Aufrechterhaltung einer günstigen Bodenbeschaffenheit gewährleistet ist.

Weiter hat es sich überraschenderweise gezeigt, dass die Aktivsubstanzen der Formel I bzw. entsprechende Mittel ausser vorteilhaften wuchsregulierenden Eigenschaften zusätzlich ein für praktische Bedürfnisse sehr günstiges Mikrobizidspektrum aufweisen. Deshalb liegt ein weiteres Einsatzgebiet von Verbindungen der Formel I in der Bekämpfung von schädlichen Mikroorganismen, vor allem von phytopathogegen Pilzen. So besitzen die Verbindungen der Formel I eine für praktische Bedürfnisse sehr günstige kurative, präventive und systemische Wirkung zum Schutz von Pflanzen, insbesondere Kulturpflanzen, ohne diese nachteilig zu beeinflussen.

Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Bortrytis, Helminthosporium, Fusarium, Septoria, Cercospora und Alternaria). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehrereb hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorhum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer):oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover crops), die einer Erosion oder Austrocknung des Bodens entgegenwirken oder Bodenbedeckungen wie sie in Baum- und Staudenkulturen (Obstplantagen, Hopfenkulturen, Maisfeldern, Weingärten usw.) erwünscht sind.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein, Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach dem Befallsdruck für den entsprechenden Erreger (Pilzsorte) oder der Art der über den Erdboden durch das Wurzelwerk in die Pflanzen gelangen (systemische Wirkung), indem man den Standort der Pflanzen mit einer flüssigen Zubereitung tränkt oder die Substanzen in fester Form in Verbindungen der Formel I können aber auch auf Samenkörner aufgebracht werden (Coating), indem man die Körner entweder mit einer flüssigen Zubereitung des Wirkstoffs tränkt oder sie mit einer festen Zuberei-Applikationsarten möglich, so z.B. die gezielte Behandlung der Pflanzenstengel oder der Knospen.

Die Verbindungen der Formel I werden dabei in unveränderter Form oder vorzugsweise zusammen mit den in

der Formulierungstechnik üblichen Hilfsmitteln eingesetzt und werden daher z.B. zu Emulsionskonzentraten, streichfähigen Pasten, direkt versprühbaren oder verdünnbaren Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha; bevorzugt 100g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, ~~wie z.B. mit~~ Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclonexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegegenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders geeignete Zusatzstoffe sind ferner Phospholipide.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierhier gehören auch die Salze der Schwefelsäureester und Sulfonsäuren von Fettalkohol-Ethylenoxyd-Addukten. Die sulfonierten Benzimi— dazolderivate enthalten, vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglycolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das

Stearyltrimethylammoniumchlorid oder das Benzyldi(2-chlorethyl)ethylammonium-bromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ringwood New Jersey, 1980
Sisely and Wood, "Encyclopedia of Surface Active Agents", Chemical Publishing Co., Inc. New York, 1980.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben. Prozente und Teile beziehen sich auf das Gewicht. RT bedeutet Raumtemperatur, h steht für Stunde, min für Minute, DMSO fur Dimethylsulfoxid, THF für Tetrahydrofuran, DMF für Dimethylformamid.

**Herstellungsbeispiele für Ausgangsprodukte:**

**Beispiel A:** Herstellung von

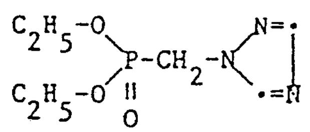

**Diethyl-(1H-1,2,4-triazol-1-yl)-methylphosphonat**

a) Zur Dispersion von 17,6 g (0,1 Mol) Kalium-diethylphosphit in 100 ml Toluol lässt man bei 90° 11,7 g (0,1 Mol) frisch hergestelltes 1-Chlormethyl-1H-1,2,4-triazol zutropfen. Nach Beendigung der exothermen Reaktion wird das ausgefallene Kaliumchlorid abgetrennt und das Filtrat eingedampft. Die Chromatographie des Rückstands mit Ethylacetat an Kieselgel ergibt 2,1 g(9,6%) gelbes Oel. Durch Kugelrohrdestillation dieses Oels erhält man Diethyl-(1H-1,2,4-triazol-1-yl)-methylphosphonat als farbloses Oel, Kp. 130°/0,11 mb.

$^1$H-NMR ($CD1_3$, TMS): $\delta$= 8,4 (s; 1H, Triazol 5-CH); 8,07 (s; 1H, Triazol 3-CH); 4,75 (d, $J_{PCH}$ = 13 Hz; 2H, P-$CH_2$); 4,25 (2q, J = Hz; 4H, $OCH_2$) und 1,5 (t, J = Hz; 6H, $CH_3$) ppm.

$^{31}$P-NMR ($CD_3OD$, $H_3PO_4$):$\delta$ = +17,12 ppm.

b) Eine Mischung aus 18,2 g (0,155 Mol) 1-Chlormethyl-1H-1,2,4-triazol, 27,2 g (0,17 Mol) Natriumdiethylphosphit und 200 ml Acetonitril wird für 2 h auf 60° erwärmt und anschliessend für 16 h unter Rückfluss erhitzt. Nach Abtrennen des Niederschlages wird die Lösung eingedampft und fraktioniert destilliert. Man erhält 19,1 g (56,5%) Diethyl-(1H-1,2,4-triazol-1-yl)-methylphosphonat als farbloses Oel. Kp. 120-129°/0,053 mb.

$C_7H_{14}N_3O_3P$ (219,2)
Ber: C 38,36%; H 6,44%; N 19,17%;
Gef: C 38,37%; H 6,60%; N 19,76%.

**Herstellungsbeispiele für Endprodukte**

**Beispiel 1:** Herstellung von

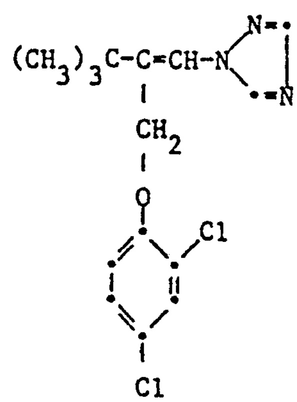

**1-(1H-1,2,4-Triazol-1-yl)-2-(2,4-dichlorphenoxymethyl)-3,3-dimethyl – buten**

100 ml Dichlormethan und 10 ml Thionylchlorid werden unter Rühren auf -30° abgekühlt und portionsweise mit 17,5 g (0,05 Mol) 1-(1H-1,3,4-Triazolyl)-2-(2,4-dichlorphenoxymethyl)-3,3-dimethyl-butanol versetzt. Man rührt das Reaktionsgemisch 2 h und lässt anschliessend eine Lösung von 15 ml Pyridin in 30 ml Dichlormethan zutropfen und rührt die Mischung ca. 12 h bei RT. Die klare Lösung wird im Vakuum eingedampft, mit Eis versetzt, mit Natriumcarbonat alkalisch eingestellt und mit Diethylether extrahiert. Die vereinigten Extrakte werden über Natriumsulfat getrocknet, filtriert und eingedampft. Der gelbliche, ölige Rückstand wird säulenchromatographisch (Silicagel/Essigsäureethylester) gereinigt. Ausbeute 75% der Theorie.

**Beispiel 2:** Herstellung von

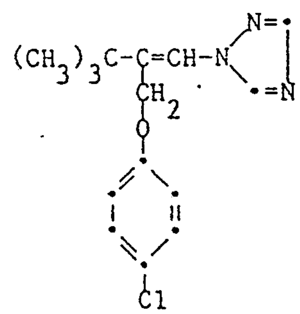

**1-(1H-1,2,4-Triazol-1-yl)-2-(4-chlorphenoxymethyl)-3,3-dimethyl-buten**

Es werden 15 g Natriumhydrid in Form einer 55%igen Oeldispersion unter Schutzgasatmosphäre (Stickstoff) in 150 ml 1,2-Dimethoxyethan (DME) vorgelegt. Dazu lässt man bei RT eine Lösung von 69 g (0,315 Mol) Diethyl-(1H-1,2,4-triazol-1-yl)methylphosphonat, 68 g (0,3 Mol) 2,2-Diemthyl-4-(4-chlorphenoxy)-butanon-3 und einer Spatelspitze 15-Krone-5 in 500 ml DME zutropfen, rührt das Reaktionsgemisch ca. 10 h, lässt Eiswasser zutropfen und extrahiert mit Methylenchlorid. Die vereinigten Extrakte werden mit Wasser gewaschen, über Natriumsulfat getrocknet, filtriert und eingedampft. Der ölige Rückstand wird säulenchromatographisch (Kieselgel/Toluol-Cyclohexan-Diethylether-Dichlormethan 4:2:2:2) gereinigt, wobei das Produkt als gelbes Oel anfällt.

$^1$H-NMR ($CDCl_3$, TMS): $\delta$ = 8.4 (s; 1H, Triazol 5-CH); 8.0 (s; 1H, Triazol 3-CH); 7.4-6.8 (m; 5H, Aryl C-H und Olefin C-H); 4.5 (s; 2H, $CH_2$-0); 1.2 (s, 9H, tert.-Butyl).

Auf analoge Weise werden auch die nachfolgenden Produkte der Formel I sowie die genannten Zwischenprodukte hergestellt. Sofern nicht speziell hervorgehoben, fällt das Produkt als E/Z-Isomerengemisch an, so dass die genannte physikalische Konstante für das Isomerengemisch gilt.

**Tabelle 1:** Verbindungen der Formel

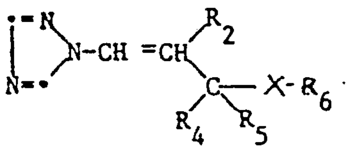

| Verb. Nr. | $R_2$ | $R_4$ | $R_5$ | $R_6$ | X | Physik. Konst. (°C) |
|---|---|---|---|---|---|---|
| 1.1 | $C_4H_9$-t | H | H | $C_6H_4Cl(4)$ | O | Isomeren Gemisch: Oel; E-Isomeres Fp. 48-50°; Z-Isomeres F. 82-86° |
| 1.2 | $C_4H_9$-t | H | H | $C_6H_3Cl_2(2,4)$ | O | Oel |
| 1.3 | $C_4H_9$-t | H | H | $C_6H_4F(4)$ | O | Isomeren Gemisch: Harz; E-Isomeres Fp. 68-70°; Z-Isomeres Fp. 79-81° |
| 1.4 | $C_4H_9$-t | H | H | $C_6H_4CF_3(3)$ | O | |
| 1.5 | $C_4H_9$-t | H | H | $C_6H_3Cl(2)Br(4)$ | O | |
| 1.6 | $C_4H_9$-t | H | H | $C_6H_5$ | O | |
| 1.7 | $C_4H_9$-t | H | H | β-Naphthyl | O | |
| 1.8 | $C_4H_9$-t | H | H | 4-Biphenyl | O | |
| 1.9 | $C_4H_9$-t | H | H | 4-Benzylphenyl | O | |
| 1.10 | $C_4H_9$-t | H | H | 4-Phenoxyphenyl | O | |
| 1.11 | $C_4H_9$-t | H | H | 4-Benzyloxyphenyl | O | |
| 1.12 | $C_4H_9$-t | H | H | $C_6H_4Cl(4)$ | S | |
| 1.13 | $C_5H_{11}$-n | H | H | $C_6H_4Cl(4)$ | O | $n_D^{25} = 1.5428$ |
| 1.14 | $C_4H_9$-t | H | H | $C_5H_4F(4)$ | O | Smp. 68-70° |
| ~~1.15~~ | ~~$CH_3$~~ | ~~H~~ | ~~H~~ | ~~$CH_3$~~ | ~~O~~ | ~~Oel~~ |
| 1.16 | $CH_3$ | H | H | Biphenyl | O | |
| 1.17 | $CH_3$ | H | H | $C_6H_3Cl(2)Br(4)$ | O | |

**Tabelle 1:** (Fortsetzung)

| Verb.Nr. | $R_2$ | $R_4$ | $R_5$ | $R_6$ | X | Physik. Konstante |
|---|---|---|---|---|---|---|
| 1.18 | $C(CH_3)_2H$ | H | $C_2H_5$ | $C_6H_4Cl(4)$ | O | $n_D^{25} = 1.5418$ |
| 1.19 | $C_4H_9$-t | H | $C_2H_5$ | $C_6H_4Cl(4)$ | O | $n_D^{35} = 1.5360$ |
| 1.20 | $C_4H_9$-t | H | $C_3H_7$-n | $C_6H_4Cl(4)$ | O | $n_D^{35} = 1.5335$ |
| 1.21 | $C_4H_9$-t | H | $C_4H_9$-n | $C_6H_4Cl(4)$ | O | $n_D^{35} = 1.5190$ |
| 1.22 | $C(CH_3)_2H$ | H | $C_3H_7$-n | $C_6H_4Cl(4)$ | O | $n_D^{35} = 1.5388$ |
| 1.23 | $C(CH_3)_2H$ | H | $CH_3$ | $C_6H_4Cl(4)$ | O | $n_D^{35} = 1.5460$ |
| 1.24 | $C(CH_3)_2H$ | H | $C_2H_5$ | $C_6H_4CH_3(4)$ | O | $n_D^{35} = 1.5288$ |
| 1.25 | $(CH_2)_8CH_3$ | H | $C_2H_5$ | $C_6H_4Cl(4)$ | O | $n_D^{36} = 1.5170$ |
| 1.26 | $C_4H_9$-t | H | $CH_3$ | $C_6H_4F(4)$ | O | $n_D^{36} = 1.5191$ |
| 1.27 | $C_4H_9$-t | H | $CH_3$ | $C_6H_4Cl(4)$ | O | $n_D^{36} = 1.5410$ |
| 1.28 | $C(CH_3)_2H$ | H | $C_2H_5$ | $C_6H_3CH_3(2)Cl(4)$ | O | $n_D^{36} = 1.5289$ |
| 1.29 | $(CH_2)_4CH_3$ | H | $C_2H_5$ | $C_6H_4Cl(4)$ | O | $n_D^{36} = 1.5261$ |
| 1.30 | $C_4H_9$-t | H | $C_2H_5$ | $C_6H_4F(4)$ | O | $n_D^{36} = 1.5088$ |

**Tabelle 1:** (Fortsetzung)

| Verb.Nr. | $R_2$ | $R_4$ | $R_5$ | $R_6$ | X | Physik. Konstante |
|---|---|---|---|---|---|---|
| 1.31 | $C(CH_3)_2H$ | H | $C_2H_5$ | $C_6H_4Br(4)$ | O | $n_D^{36} = 1.5502$ |
| 1.32 | $C_4H_9$-t | H | $C_2H_5$ | $C_6H_4CF_3(4)$ | O | $n_D^{37} = 1.4940$ |
| 1.33 | $C_4H_9$-t | H | $C_2H_5$ | $C_6H_4Cl(2)$ | O | $n_D^{36} = 1.5370$ |
| 1.34 | $CH_2CH(CH_3)_2$ | H | $C_2H_5$ | $C_6H_9Cl(4)$ | O | Sdp. 155°/5.10$^{-3}$mbar |
| 1.35 | 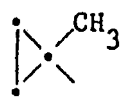 | H | H | $C_6H_4Cl(4)$ | O | |
| 1.36 | 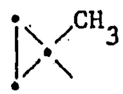 | H | H | $C_6H_3Cl_2(2,4)$ | O | |
| 1.37 | $C_6H_3Cl_2(2,4)C(CH_3)_2$ | H | H | $C_6H_5$ | O | |
| 1.38 | $C_6H_3Cl_2(2,4)C(CH_3)_2$ | H | H | $C_6H_4F(4)$ | O | |
| 1.39 | $C_6H_3Cl_2(2,4)C(CH_3)_2$ | H | H | $C_6H_4Cl(4)$ | O | |

| Verb. Nr. | $R_2$ | $R_4=R_5$ | $R_6$ | X | Physik. Konstante |
|---|---|---|---|---|---|
| 1.140 | $C_4H_9$-t | $CH_3$ | 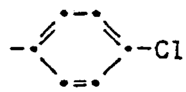 | o | |
| 1.141 | $C_4H_9$-t | $CH_3$ | $C_6H_4Cl(4)$ | o | |
| 1.142 | 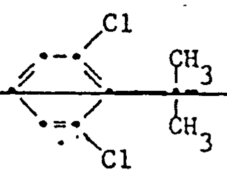 | H | 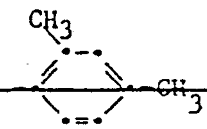 | – | |
| 1.143 | $CH_3$ | H | $-CH_2CH=CH_2$ | – | |
| 1.144 | $CH_3$ | H | 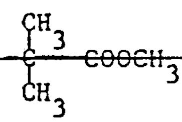 | – | |
| 1.145 | $CH_3$ | H | 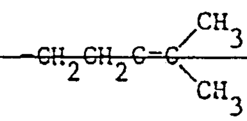 | – | |
| 1.146 | $CH_3$ | H | 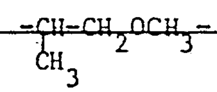 | – | |
| 1.147 | $CH_3$ | H | 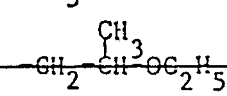 | – | |
| 1.148 | 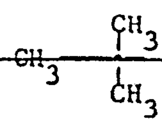 | H | 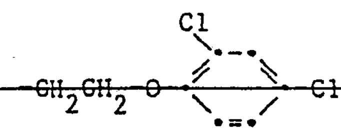 | – | |
| 1.149 | 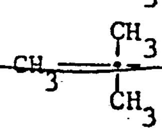 | H | 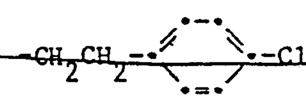 | – | |
| 1.150 | 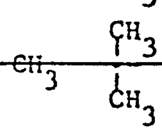 | H | 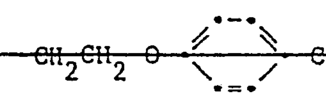 | – | |
| 1.151 | 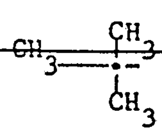 | H | $-CH_2CH_2OC_2H_5$ | – | |
| 1.152 | $C(CH_3)_2H$ | H | $C_6H_4Cl(4)$ | o | $n_D^{25} = 1.5501$ |

| Verb. Nr. | $R_2$ | $R_4=R_5$ | $R_6$ | X | Physik. Konst. |
|---|---|---|---|---|---|
| 1.153 | $C(CH_3)_2H$ | H | $C_6H_4F(4)$ | O | |
| 1.154 | $C(CH_3)_2H$ | H | $C_6H_4Br(4)$ | O | |
| 1.155 | $C(CH_3)_2H$ | H | $C_6H_4CH_3(4)$ | O | |
| 1.156 | $C(CH_3)_2CH_2CH_3$ | H | $C_6H_4Cl(4)$ | O | |
| 1.157 | $C(CH_3)_2CH_2CH_3$ | H | $C_6H_4F(4)$ | O | |
| 1.158 | $C(CH_3)_2CH_2CH_3$ | H | $C_6H_4Br(4)$ | O | |
| 1.159 | $C(CH_3)_2CH_2CH_2$ | H | $C_6H_4CH_3(4)$ | O | |
| 1.160 | $C(CH_3)HCH_2CH_2CH_3$ | H | $C_6H_4Cl(4)$ | O | $n_D^{25}$= 1.5459 |
| 1.161 | $C(C_2H_5)HC_4H_9$-n | H | $C_6H_4Cl(4)$ | O | Sdp.155-160°/ 0.002 mbar |
| 1.162 | $C(C_2H_5)HC_4H_9$-n | H | $C_6H_4CH_3(4)$ | O | Sdp.150-155°/ 0.004 mbar |
| 1.163 | $C(CH_3)HCH_2CH_3$ | H | $C_6H_4CH_3(4)$ | O | |
| 1.164 | $C(CH_3)HCH_2CH_3$ | H | $C_6H_3Cl_2(2,4)$ | O | |
| 1.165 | $C(CH_3)_2CH_2CH_3$ | H | $C_6H_3Cl_2(2,4)$ | O | |
| 1.166 | $C(CH_3)_2H$ | H | $C_6H_3Cl_2(2,4)$ | S | |
| 1.167 | $C(CH_3)_2H$ | H | $C_6H_3Cl_2(2,4)$ | O | |
| 1.168 | $C(CH_3)_2H$ | $CH_3$ | $C_6HF(4)$ | O | |

Formulierungsbeispiele für flüssige Wirkstoffe der Formel I

(% = Gewichtsprozent)

| 3. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykol-ehter (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 4. Lösungen | a) | b) | c) | d) |
|---|---|---|---|---|
| Wirkstoff aus den Tabellen | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| 5. Granulate | a) | b) |
|---|---|---|
| Wirkstoff aus den Tabellen | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 6. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

Formulierungsbeispiele für feste Wirkstoffe der Formel I

(% = Gewichtsprozent)

| 7. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykoläther (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

8. Emulsions-Konzentrat

| | |
|---|---|
| Wirkstoff aus den Tabellen | 10% |
| Octylphenolpolyethylenglykolether (4-5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 9. Stäubemittel | a) | b) |
|---|---|---|
| Wirkstoff aus den Tabellen | 5% | 8% |
| Talkum | 95% | - |
| Kaolin | - | 92% |

Man erhält anwendungsfertige Stäubemittel, indem der Wirkstoff mit den Träger vermischt und auf einer geeigneten Mühle vermahlen wird.

10. Extruder Granulat

| | |
|---|---|
| Wirkstoff aus den Tabellen | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

11. Umhüllungs-Granulat

| | |
|---|---|
| Wirkstoff aus den Tabellen | 3% |
| Polyethylenglykol (M G 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

12. Suspensions-Konzentrat

| | |
|---|---|
| Wirkstoff aus den Tabellen | 40% |
| Ethylenglykol | 10% |
| Nonylphenolpolyethylenglykolether (15 Mol Ethylenoxid) | 6% |
| N-Lingninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Biologische Beispiele:**

**Beispiel 13: Wirkung gegen Puccinia graminis auf Weizen**

**a) Residual-protektive Wirkung**

Weizenpflanzen wurden 6 Tage nach der Aussaat mit einer aus Spritzpulver des Wirkstoffes hergestellten Spritzbrühe (0,06% Aktivsubstanz) besprüht. Nach 24 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rotpustelnentwicklung erfolgte 12 Tage nach der Infektion.

**b) Systemische Wirkung**

Zu Weizenpflanzen wurde 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Ureidosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Verbindungen aus den Tabellen 1 zeigten gegen Puccinia-Pilze eine gute Wirkung. Unbehandelte aber infizierte Kontrollpflanzen zeigten einen Puccinia-Befall von 100%. Insbesondere Substanzen aus Anspruch 3 zeigten eine ausgeprägte Aktivität gegen Puccinia-Pilze, vor allem Verbindung Nr. 1.1.

**Beispiel 14: Wirkund gegen Cercospora arachidicola auf Erdnusspflanzen**

**Residual-protektive Wirkung**

10-15 cm hohe Erdnusspflanzen wurden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftraten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen, die mit Wirkstoffen aus den Tabellen 1 behandelt wurden, einen stark reduzierten Cercospora-Befall. So verhinderten die Verbindungen aus Anspruch 3 den Cercosporabefall. In besonders hohem Ausmass reduzierte Verbindung Nr. 1.1 den Befall, nämlich auf 0 bis 5%.

**Beispiel 15: Wirkung gegen Erysiphae graminis auf Gerste**

**a) Residual-protektive Wirkung**

Ca. 8 cm hohe Gerstenpflanzen wurden mit einer aus Spritzpulver des Wirkstoffes hergestellten Spritzbrühe (0,02% Aktivsubstanz) besprüht. Nach 3-4 Stunden wurden die behandelten Pflanzen mit Konidien des Pilzes bestäubt. Die infizierten Gerstenpfkanzen wurden in einem Gewächshaus bei ca. 22°C aufgestellt und der Pilzbefall nach 10 Tagen beurteilt.

**b) Systemische Wirkung**

Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100%. Unter anderen Verbindungen aus den Tabellen 1

hemmten die Verbindungen Nr. 1.1 und 1.2, aber auch weitere Vertreter, zeigen sich besonders wirksam gegen Venturia-Pilze.

**Beispiel 16: Residual-protektive Wirkung gegen Venturia inaequalis auf Apfeltrieben**

Apfelstecklinge mit 10-20 cm langen Frischtrieben wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt.

**Beispiel 17: Wirkung gegen Botrytis cinerea auf Bohnen Residual protektive Wirkung**

Ca. 10 cm hohe Bohnen-Pflanzen wurden mit einer aus Spritzpulver des Wirkstoffes hergestellten Spritzbrühe (0,02% Aktivsubtanz) besprüht. Nach 48 Stunden wurden die behandelten Pflanzen mit einer Konidiensuspension des Pilzes infiziert. Nach einer Inkubation der infizierten Pflanzen während 3 Tagen bei 95.100% relativer Luftfeuchtigkeit und 21°C erfolgte die Beurteilung des Pilzbefalls. Die Verbindungen aus den Tabellen 1 hemmten in vielen Fällen die Pilzinfektion sehr stark. Bei einer Konzentration von 0,02% erweisen sich z.B. die Verbindungen Nr. 1.1, 1.2 als ausgesprochen aktiv gegen Botrytis-Pilze. Sie hemmen z.T. den Befall vollständig.

**Beispiel 18: Wuchshemmung bei Getreide**

In Kunststofftöpfen mit sterilisierter Erde wurden die Getreidearten Hordeum vulgare (Sommergerste) und Secale (Sommerroggen) im Gewächshaus angesät und nach Bedarf bewässert. Die Sprösslinge wurden ca. 21 Tage nach der Aussaat mit der wässrigen Spritzbrühe eines Wirkstoffes der Formel I besprüht. Die Wirkstoffmenge betrug umgerechnet 0,5 bzw. 2,5 kg Aktivsubstanz pro Hektar. 10 und 21 Tage nach Applikation wurde das Wachstum des Getreides beurteilt. Hierbei konnte festgestellt werden, dass Getreidepflanzen, die mit Wirkstoffen der Formel I behandelt worden waren, im Vergleich zu unbehandelten Kontrollpflanzen Wuchsreduktionen aufweisen.

**Beispiel 19: Wuchshemmung bei Gräsern**

In Kunststoffschalen mit Erde-Torf-Sand-Gemisch (6:3:1) wurden die Gräser Lolium perenne, Poa pratensis, Festuca orina und Cynodon dactylon im Gewächshaus angesät und nach Bedarf bewässert. Die aufgelaufenen Gräser wurden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge betrug umgerechnet 0,5 bzw. 2,5 kg Aktivsubstanz pro Hektar. 10 und 21 Tage nach Applikation wurde das Wachstum der Gräser beurteilt, dabei zeigte es sich, dass die erfindungsgemässen Wirkstoffe aus den Tabellen 1 Wuchshemmungen bewirkten.

**Beispiel 20: Ertragssteigerung an Sojabohnen**

In Kunststoffbehältern mit einem Erde-Torf-Sandgemisch im Verhältnis 6:3:1 wurden Sojabohnen der Sorte "Hark" angesät und in eine Klimakammer gegeben. Durch optimale Temperaturwahl, Beleuchtung, Düngerzugabe und Bewässerung entwickelten sich die Pflanzen nach ca. 5 Wochen in dem 5-6 Trifola-Blattstadium. Zu diesem Zeitpunkt wurden die Pflanzen mit der wässrigen Brühe eines Wirkstoffes der Formel I bis zur guten Benetzung besprüht. Die Wirkstoffkonzentration betrug bis zu 500 ppm Aktivsubstanz. Die Auswertung erfolgte ca. 5 Wochen nach der Applikation des Wirkstoffs. Im Vergleich zu unbehandelten Kontrollpflanzen bewirkten die erfindungsgemässen Wirkstoffe der Formel I eine merkliche Erhöhung der Anzahl und des Gewichts der geernteten Schoten. Als besonders wirksam erwiesen sich die Verbindungen aus den Tabellen 1. Insbesondere die Verbindungen 1.1, 1.13, 1.14, 1.34, 1.160, 1.161 und 1.162 trugen zu einer Ertragssteigerung bei. Als besonders wirksam erwies sich die Verbindung Nr. 1.21.

**Beispiel 21: Wuchshemmung bei Bodenbedecker-Pflanzen (Cover-Crops)**

In Kunststoffschalen mit Erde-Torf-Sand-Gemisch (1:1:1) werden Testpflanzen der Sorte Psophocarpus palustris und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagestemperatur von 27°C und einer Nachttemperatur von 21°C, bei einer mittleren Lichtdauer von 14 h (6000 Lux) und einer Luftfeuchtigkeit von 70% statt. Die Testpflanzen werden auf eine Höhe von ca. 15 cm. zurückgeschnitten und 7 Tage nach dem Zurückschneiden mit einer 8pritzbrühe des Wirkstoffes (umgerechnet 0,3 bzw. 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass viele Verbindungen aus der Tabelle 1 eine deutliche Wuchshemmung der Bodenbedecker-Pflanzen auslösen.

**Beispiel 22: Seneszenzhemmung bei Getreidepflanzen**

Im Gewächshaus wird Sommerweizen der Sorte "Svenno" in Töpfen mit Komposterde angesät und ohne spezielle klimatische Anforderungen gezogen. Ca. 10 Tage nach dem Auflaufen werden 10 bis 12 cm lange Primärblätter abgeschnitten und einzeln in Reagenzgläser mit 10 ml einer Wirkstoffsuspension (1,25 bis 10 ppm Aktivsubstanz) gegeben. Die Reagenzgläser werden in einem klimatisierten Raum bei 23°C, 70% relativer Luftfeuchtigkeit aufgestellt und täglich durchschnittlich 14 Stunden (10 000 Lux) bestrahlt. Die Beurteilung der Seneszenzhemmung erfolgt 7 Tage nach Einstellen der Blätter durch Vergleich des Vergilbungsgrades im Verhältnis zu noch frischen, grünen Blättern. Bei diesem Versuch kann beobachtet werden, dass Verbindungen aus der Tabelle 1 eine deutliche Inhibierung der Seneszenz der Testpflanzen hervorrufen, insbesondere hemmten sie das Vergilben der Blätter im Versuchszeitraum um mehr als 80%.

**Patentansprüche:**

1. Verbindungen der Formel I

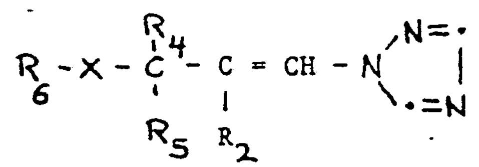

(I),

worin

$R_2$ für $C_1$-$C_{12}$-Alkyl, $C_3$-$C_8$-Cycloalkyl, durch gegebenenfalls substituiertes Phenyl, $C_1$-$C_4$-Alkoxycarbonyl oder $C_2$-$C_4$-Alkenyl substituiertes $C_1$-$C_4$-Alkyl oder ein- bis vierfach durch $C_1$-$C_4$-Alkyl substituiertes $C_3$-$C_8$-Cycloalkyl steht;

$R_4$ und $R_5$ unabhängig voneinander für Wasserstoff oder $C_1$-$C_4$-Alkyl stehen;

X Sauerstoff oder Schwefel bedeutet;

$R_6$ für einen unsubstituierten oder ein- oder mehrfach substituierten Rest steht, ausgewählt aus der Reihe: Phenyl, Naphthyl, Biphenyl, Benzylphenyl, Benzoxyphenyl, Phenoxyphenyl und Aralkyl, wobei die Substituenten ausgewählt sind aus der Reihe: Halogen, Cyano, $C_1$-$C_4$-Alkyl, $C_1$-$C_5$-Alkoxy, $C_1$-$C_5$-Haloalkoxy, $C_1$-$C_3$-Haloalkyl oder Nitro,

bedeuten; unter Einschluss ihrer Säureadditionssalze, quaternären Azoliumsalze und Metallkomplexe.

2. Eine Verbindung der Formel I nach Anspruch 1, ausgewählt aus der Reihe:

1-(1H-1,2,4-Triazol-1-yl)-2-(4-chlorphenoxymethyl)-3,3-dimethylbuten,

1-(1H-1,2,4-Triazol-1-yl)-2-(iso-propyl)-3-(4-chlorphenoxy)-penten,

1-(1H-1,2,4-Triazol-1-yl)-2-(tert.-butyl)-3-(4-fluorphenoxy)-buten,

3. Verfahren zur HerstellungvonAzolyl-derivaten der Formel I nach Anspruch 1, dadurch gekennzeichnet, dass man entweder nach Variante i ein Arylketon der Formel II

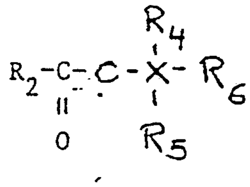 (II).

worin $R_2$, $R_4$, $R_5$, $R_6$ und X die unter Formel I angegebenen Bedeutungen haben, mit einem 1H-Azol-1-yl-methylphosphonat der Formel III,

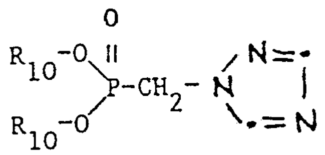 (III)

worin die Reste $R_{10}$ unabhängig voneinander Phenyl oder $C_1$-$C_4$-Alkyl bedeuten, oder mit einem Phosphoniumsalz der Formel IV,

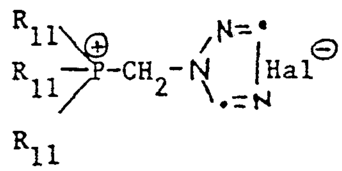 (IV)

worin die Reste $R_{11}$ unabhängig voneinander Phenyl doer gegebenenfalls durch Hydroxyl substituiertes $C_1$-$C_4$-Alkyl bedeuten, und Hal für Chlor oder Brom steht in einem reaktionsinerten Lösungsmittel in Gegenwart einer starken Base umsetzt, oder gemäss Variante ii, indem man direkt aus einer Verbindung der Formel V,

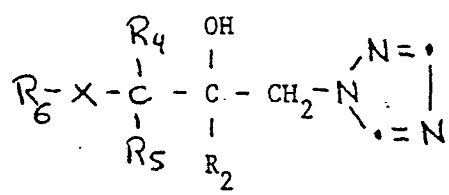 (V)

worin $R_2$, $R_4$, $R_5$, $R_6$ und X die unter Formel I angegebenen Bedeutungen haben, mit einem wasserentziehenden Mittel Wasser abspaltet, oder indem man die Verbindung der Formel V durch Austausch der freien Hydroxylgruppe gegen eine der üblichen Abgangsgruppen A zuerst in eine Verbindung der Formel VI überführt

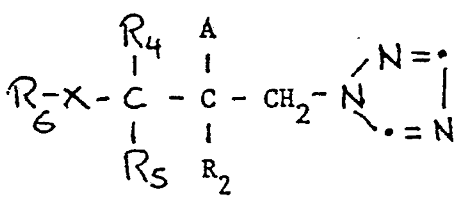 (VI)

und aus VI dann durch Zusatz einer Base die hydrierte Abgangsgruppe H-A abspaltet; oder indem man nach Variante iii ein Chlor-olefinderivat der Formel VII,

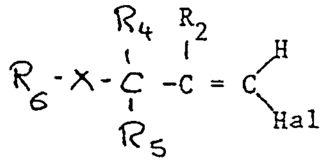

(VII),

worin $R_2$, $R_4$, $R_5$, $R_6$ und X die unter Formel I angegebenen Bedeutungen haben, und der Substituent Hal für Halogen steht, in der Schmelze bei +150° bis +350°C und gegebenenfalls unter Druck mit überschüssigem 1H-1,2,4-Triazol umsetzt; oder indem man nach Variante iv eine Verbindung der Formel IX,

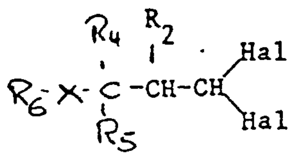

(IX)

worin $R_2$, $R_4$, $R_5$, $R_6$ und X die unter Formel I angegebenen Bedeutungen haben, und die Substituenten Hal für Halogen stehen, in einem dipolaren aprotischen Lösungsmittel und gegebenenfalls in Gegenwart einer Base bei +40° bis +180°C oder in der Schmelze bei +150° bis +350°C und gegebenenfalls in Gegenwart einer Base mit überschüssigem Azol der Formel VIII umsetzt.

4. Mittel zur Regulierung des Pflanzenwachstums, dadurch gekennzeichnet, dass es als aktive Komponente eine Verbindung der Formel I gemäss Anspruch 1 enthält.

5. Mittel gemäss Anspruch 4, dadurch gekennzeichnet, dass es als mindestens eine aktive Komponente eine Verbindung der Formel I gemäss Anspruch 2 enthält.

6. Verfahren zur Regulierung des Pflanzenwachstums, dadurch gekennzeichnet, dass man eine gemäss Anspruch 1 definierte Verbindung der Formel I auf die Pflanze oder deren Standort appliziert.

7. Verwendung von Verbindungen der Formel I gemäss Anspruch 1 zur Regulierung des Pflanzenwachstums.

8. Verwendung gemäss Anspruch 7 zur Wuchshemmung bei Bodenbedeckerpflanzen.

9. Verwendung gemäss Anspruch 7 zur Wuchsregulierung bei Leguminosen im Sinne einer Ertragssteigerung.

**Claims:**

1. A compound of the formula I

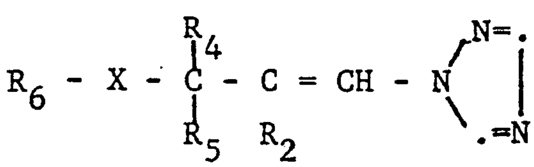

(I),

wherein

$R_2$ is $C_1$-$C_{12}$alkyl, $C_3$-$C_8$cycloalkyl, $C_1$-$C_4$alkyl substituted by unsubstituted or substituted phenyl, $C_1$-$C_4$alkoxycarbonyl or $C_2$-$C_4$alkenyl, or is $C_3$-$C_8$cycloalkyl which is substituted by 1 to 4 $C_1$-$C_4$alkyl groups,

$R_4$ and $R_5$, each independently of the other, are hydrogen or $C_1$-$C_4$alkyl,

X is oxygen or sulfur,

$R_6$ is a radical selected from the group consisting of phenyl, naphthyl, biphenyl, benzylphenyl, benzoxyphenyl, phenoxyphenyl and aralkyl, which radical is unsubstituted or substituted by one or more of halogen, cyano $C_1$-$C_4$alkyl, $C_1$-$C_5$alkoxy, $C_1$-$C_5$haloalkoxy, $C_1$-$C_3$haloalkyl or nitro,

or an acid addition salt, quaternary azolium salt or metal complex thereof.

2. A compound of the formula 1 according to claim 1 selected from the group consisting of: 1-(lR-1,2,4-triazol-1-yl)-2-(4-chlorophenoxymethyl)-3,3-dimethylbutene, 1-(lR-1,2,4-triazol-1-yl)-2-(isopropyl)-3-(4-chlorophenoxy)-pentene, 1- (lR-1,2,4-triazol-1-yl)-2-(tert-butyl)-3-(4-fluorophenoxy)-butene.

3. A process for the preparation of an azolyl derivative of the formula 1 according to claim 1, which process comprises either reacting, according to variant (i), an arylketone of the formula II

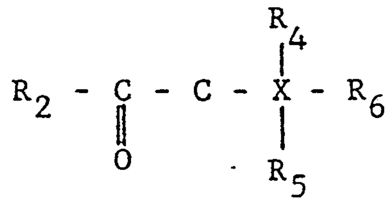 (II)

wherein $R_2$, $R_4$, $R_5$, $R_6$ and X are as defined for formula I, with a IR-azol-1-yl-methylphosphonate of the formula III

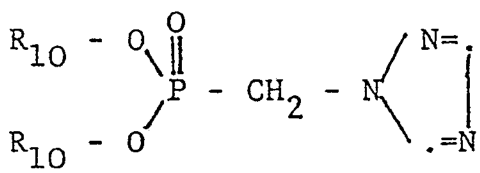 (III)

wherein each of the radicals $R_{10}$ independently of the other is phenyl or $C_1$-$C_4$alkyl, or with a phosphonium salt of the formula IV

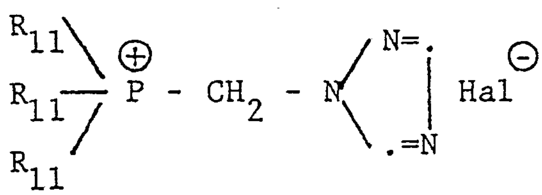 (IV)

wherein each of the radicals $R_{11}$ independently of the other is phenyl or $C_1$-$C_4$alkyl which is unsubstituted or substituted by hydroxyl, and Hal is chlorine or bromine, in an inert solvent and in the presence of a strong base; or, according to variant (ii), by removing water either direct from a compound of the formula V

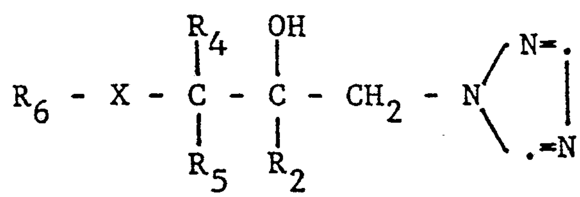 (V)

wherein $R_2$, $R_4$, $R_5$ $R_6$ and X are as defined for formula 1, with a condensing agent, or by converting the compound of the formula V first into a compound of the formula VI

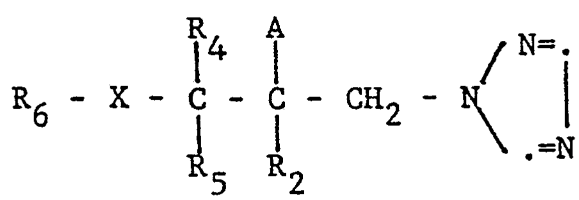 (VI)

by replacing the free hydroxyl group by a customary leaving group A, and then removing the hydrogenated leaving group R-A from the compound of formula (VI) by adding a base; or, according to variant (iii), by reacting a chloro-olefin derivative of the formula VII

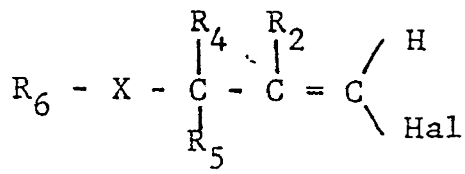

(VII)

wherein $R_2$, $R_4$, $R_5$ $R_6$ an X are as defined for formula I and Ral is halogen, in the melt in the temperature range from 150° to 50°C and optionally under pressure, with an excess of IR-1,2,4-triazole;

or, according to variant (iv), by reacting a compound of the formula IX

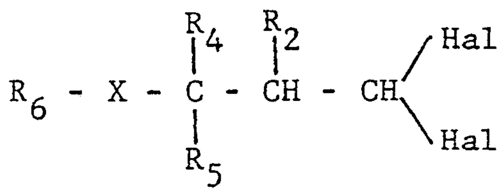

(IX)

wherein $R_2$, $R_4$, $R_5$, $R_6$ and X are as defined for formula I, and Hal is halogen, in a dipolar aprotic solvent, if desired in the presence of a base, in the temperature range from 40° to 180°C, or in the melt in the temperature range from 150° to 50°C, with an excess of azole of the formula VIII.

4. A composition for regulating plant growth, which contains as active component a compound of the formula I as claimed in claim 1.

5. A composition according to claim 4, which contains as active component at least one compound of the formula I as claimed in claim 2.

6. A method of regulating plant growth, which comprises applying to said plants or to the locus thereof an effective amount of a compound of the formula I as defined in claim 1.

7. Use of a compound of formula I according to claim 1 for regulating plant growth.

8. Use according to claim 7 for inhibiting the growth of cover crops.

9. Use according to claim 7 for inhibiting the growth of leguminosae to increase the yield.

**Revendications**

1 - Composés de formule I

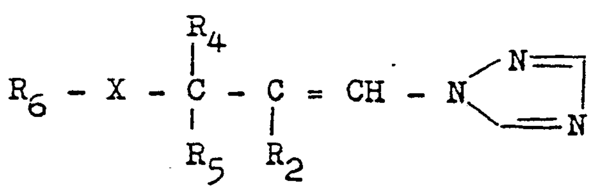

(I),

dans laquelle

$R_2$ représente un groupe alkyle en $C_1$-$C_{12}$, cycloalkyle en $C_3$-$C_8$, alkyle en $C_1$-$C_4$ substitué par un groupe phényle, (alcoxy en $C_1$-$C_4$)-carbonyle ou alcényle en $C_2$-$C_4$ éventuellement substitué, ou cycloalkyle en $C_3$-$C_8$ portant un à quatre substituants alkyle en $C_1$-$C_4$;

$R_4$ et $R_5$ représentent chacun, indépendamment l'un de l'autre, l'hydrogène ou un groupe alkyle en $C_1$-$C_4$;

X représente l'oxygène ou le soufre;

$R_6$ représente un reste non substitué ou mono- ou polysubstitué choisi dans la série: phényle, naphtyle, biphényle, benzylphényle, benzoxyphényle, phénoxyphényle et aralkyle, les substituants étant choisis dans la série: halogéno, cyano, alkyle en $C_1$-$C_4$, alcoxy en $C_1$-$C_5$, halogéno-alcoxy en $C_1$-$C_5$, halogénoalkyle en $C_1$-$C_3$ ou nitro,

y compris leurs sels formés par addition avec des acides, sels d'azolium quaternaires et complexes métalliques.

2 - Un composé de formule I selon la revendication 1 choisi dans la série:

1-(1H-1,2,4-triazole-1-yl)-2-(4-chlorophénoxyméthyl)-3,3-diméthyl-butène,

1-(1H-1,2,4-triazole-1-yl)-2-(isopropyl)-3-(4-chloro-phénoxy)-pentène,
1-(1H-1,2,4-triazole-1-yl)-2-(tert-butyl)-3-(4-fluoro-phénoxy)-butène.

3 - Procédé de préparation des dérivés azolytiques de formule I selon la revendication 1, caractérisé en ce que, selon variante i), on fait réagir une arylcétone de formule II

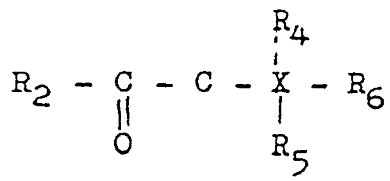 (II)

dans laquelle $R_2$, $R_4$, $R_5$, $R_6$ et X ont les significations indiquées en référence à la formule I, avec un 1H-azole-1-yl-méthylphosphonate de formule III

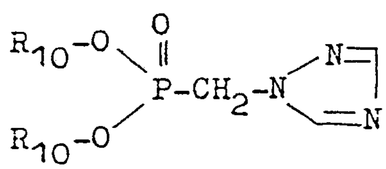 (III)

dans laquelle les symboles $R_{10}$ représentent chacun, indépendamment l'un de l'autre, un groupe phényle ou alkyle en $C_1$-$C_4$, ou avec un sel de phosphonium de formule IV,

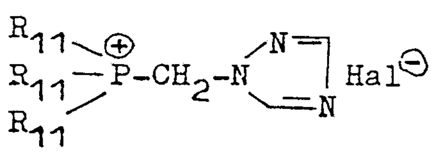 (IV)

dans laquelle les symboles $R_{11}$ représentent chacun, indépendamment les uns des autres, un groupe phényle ou alkyle en $C_1$-$C_4$ éventuellement substitué par un groupe hydroxy, et Hal représente le chlore ou le brome, dans un solvant inerte dans la réaction, en présence d'une base forte, ou bien, selon variante ii), partant directement d'un composé de formule V

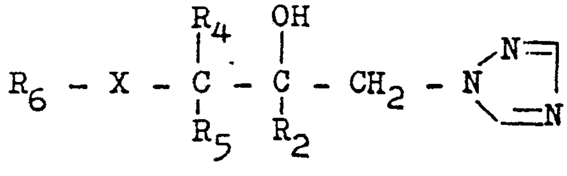 (V)

dans laquelle $R_2$, $R_4$, $R_5$, $R_6$ et X ont les significations indiquées en référence à la formule I, on élimine de l'eau à l'aide d'un agent déshydratant ou bien on convertit d'abord le composé de formule V, par échange du groupe hydroxy libre contre l'un des groupes éliminables usuels A, en un composé de formule VI

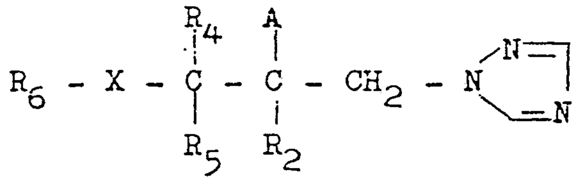

(VI)

et, par addition d'une base, on élimine du composé VI le groupe éliminable hydrogéné H-A; ou bien, selon variante iii), on fait réagir un dérivé chloro-oléfinique de formule VII

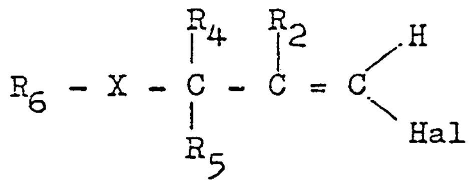

(VII)

dans laquelle $R_2$, $R_4$, $R_5$, $R_6$ et X ont les significations indiquées en référence à la formule I et Hal représente un halogène, à l'état fondu à une température de +150 à +350°C et le cas échéant on fait réagir sous pression avec un excès de 1H-1,2,4-triazole; ou bien, selon variante iv), on fait réagir un composé de formule IX

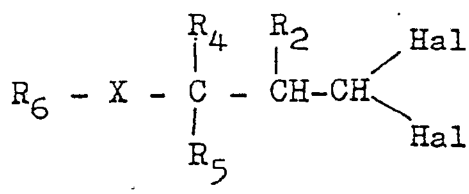

(IX)

dans laquelle $R_2$, $R_4$, $R_5$, $R_6$ et X ont les significations indiquées en référence à la formule I et Hal représente un halogène, dans un solvant aprotonique dipolaire et le cas échéant en présence d'une base, à une température de +40 à +180°C, ou bien à l'état fondu à une température de +150 à +350°C et eventuellement en présence d'une base, avec un excès de l'azole de formule VIII.

4 - Produit servant à la régulation de la croissance des végétaux, caractérisé en ce qu'il contient en tant que composant actif un composé de formule I selon la revendication 1.

5 - Produit selon la revendication 4, caractérisé en ce qu'il contient au moins un composant actif consistant en un composé de formule I selon la revendication 2.

6 - Procédé pour la régulation de la croissance des végétaux, caractérisé en ce que l'on applique sur la plante ou son habitat un composé de formule I selon la revendication 1.

7 - Utilisation des composés de formule I selon la revendication 1 pour la régulation de la croissance des vegetaux.

8 - Utilisation selon la revendication 7 pour l'inhibition de la croissance des végétaux de couvertures de sol.

9 - Utilisation selon la revendication 7 pour la régulation de la croissance des légumineuses dans le sens d'une augmentation de récolte.